# EUROPÄISCHE PATENTSCHRIFT

(11) **EP 2 417 243 B1**
(45) Veröffentlichungstag und Bekanntmachung des Hinweises auf die Patenterteilung: **05.08.2015**
(21) Anmeldenummer: 10721639.2
(22) Anmeldetag: 08.04.2010
(51) Int. Cl.: C12M 1/00, C12M 3/00, C12M 1/12, C12M 1/26

(54) **PHOTOBIOREAKTOR SOWIE VERFAHREN ZUR KULTIVATION VON BIOMASSE MITTELS PHOTOSYNTHESE**
PHOTOBIOREACTOR AND METHOD FOR CULTIVATING BIOMASS BY MEANS OF PHOTOSYNTHESIS
PHOTOBIORÉACTEURS ET PROCÉDÉ POUR LA CULTURE DE BIOMASSE PAR LA PHOTOSYNTHÈSE

(30) Priorität: 09.04.2009 DE 102009016738
(43) Veröffentlichungstag der Anmeldung: 15.02.2012
(73) Patentinhaber: Salata GmbH, 98617 Ritschenhausen (DE)
(72) Erfinder: WINKEL, Wolfram, 98631 Römhild (DE)
(74) Vertreter: Patent- und Rechtsanwälte Ullrich & Naumann
(86) Internationale Anmeldenummer: PCT/DE2010/000396
(87) Internationale Veröffentlichungsnummer: WO 2010/115412

(56) Entgegenhaltungen:
- EP-A1- 1 925 660
- WO-A2-2009/142765
- DE-A1- 19 814 253
- DE-A1- 19 958 142
- DE-U1- 29 607 285
- DE-U1-202006 013 772

## Beschreibung

Die Erfindung betrifft einen Photobioreaktor zur Kultivation von Biomasse mittels Photosynthese, insbesondere wobei die Biomasse Algen aufweist, wobei innerhalb des Bioreaktors einer wässrigen Biomasse enthaltenden Kultursuspension Licht und ggf. Kohlendioxid und/oder Nährstoffe zuführbar sind, und wobei der Photobioreaktor zumindest die folgenden Bauteile aufweist:
- einen Photosyntheseteil,
- einen System- oder Ausgleichsbehälter,
- eine Systempumpe,
- eine Regelungseinheit, und
- eine Separations- bzw. Ernteeinrichtung.

Des Weiteren betrifft die vorliegende Erfindung ein Verfahren zur Kultivation von Biomasse mittels Photosynthese, insbesondere wobei die Biomasse Algen aufweist, wobei das Verfahren die folgenden Schritte aufweist:
- Bereitstellen einer wässrigen, Biomasse enthaltenden Kultursuspension in einem Photobioreaktor,
- Kultivieren der Biomasse durch Eintragen von Licht und ggf. Kohlendioxid und/oder Nährstoffen, wobei die Suspension in dem Photobioreaktor im Kreislauf gefördert wird,
- Abtrennen und Entferner kultivierter Biomasse aus der Suspension und ggf. Entfernen von Sauerstoff und/oder sonstigen Stoffwechselprodukten der Biomasse.

Photobioreaktoren sowie Verfahren der in Rede stehenden Art sind aus der Praxis bereits bekannt, so bspw. aus der DE 198 14 253 C2 und DE 296 07 285 U1.

Durch die Kultivation von Biomasse, zumeist von Algen, lassen sich Wertstoffe wie Pharmazeutika mit Polysacchariden, polyungesättigten Fettsäuren, Farbstoffen, Vitaminen, u. a. gewinnen.

Aufgrund des hohen Anteils an Kohlenhydraten, ungesättigten Fettsäuren und Beta-Karotinen ist entsprechendes Algenmaterial auch interessant als Grundstoff von Lebensmitteln. Des Weiteren ist solches Algenmaterial hervorragend als Tierfutter, insbesondere für die Fischzucht, geeignet.

Des Weiteren lassen sich aus Algen Biogas oder Treibstoffe ("Biodiesel") herstellen, oder können diese als Biomasse verfeuert werden.

Andererseits dient entsprechend kultiviertes Algenmaterial - im Hochpreissegment - auch als Grundstoff in der kosmetischen Industrie.

Bei der Kultivation von Biomasse mittels Photosynthese stellt sich stets das Problem, dass die eingestellten Wachstumsbedingungen nicht nur dem Wachstum der eigentlich erwünschten Algenart zuträglich sind, sondern auch das Gedeihen unerwünschter Fremdorganismen wie Fremdalgen, Bakterien und/oder Pilze gefördert wird. Demzufolge ist bei dem Betrieb eines Photobioreaktors stets darauf zu achten, dass keine mikrobiellen Verunreinigungen eingeschleppt werden. Dies geschieht insbesondere beim Gasaustausch durch den Säuerstoffaustrag über eine Öffnung im Systembehälter, bei der Zuführung von Wasser und Nährmedien oder bei Arbeiten während des Betriebs der Anlage.

Des Weiteren begegnet man ganz allgemein dem Problem, dass bekannte Photobioreaktoren bezüglich der Anschaffung und dem Betrieb äußerst kostspielig sind. Die hohen Investitionskosten für eine neu aufzubauende, fest installierte Kultivationsanlage werden oft gescheut, auch wenn unternehmerseitig Interesse an einem Experimentieren mit den Möglichkeiten der Kultivation von Biomasse besteht.

Des Weiteren sind bestehende Anlagen von einem hohen Wertverlust betroffen, da diese praktisch nicht weiterveräußerbar sind. Dem steht der aufwändige Ab- und Wiederaufbau der Anlage entgegen, der eine Standortverlagerung unwirtschaftlich macht.

Der vorliegenden Erfindung liegt daher die Aufgabe zugrunde, eine Vorrichtung und ein Verfahren der eingangs genannten Art derart auszugestalten und weiterzubilden, dass eine kostengünstige und flexible Kultivation von Biomasse realisiert ist.

Erfindungsgemäß wird die voranstehende Aufgabe zunächst hinsichtlich eines Photobioreaktors zur Kultivation von Biomasse mit den Merkmalen des Patentanspruchs 1 gelöst. Danach ist ein Photobioreaktor der eingangs genannten Art dadurch gekennzeichnet, dass der Photobioreaktor als autarkes Gesamtsystem ausgelegt ist, und dass das Gesamtsystem in einem Container oder in einem die entsprechenden Maße aufweisenden und stapelbaren Rahmen angeordnet ist.

Vorteilhafte Ausgestaltungen und Weiterbildungen des erfindungsgemäßen Photobioreaktors sind den nachgeordneten Patentansprüchen 2 bis 11 entnehmbar.

In erfindungsgemäßer Weise ist erkannt worden, dass der Photobioreaktor als autarke Anlage eine größtmögliche Flexibilität aufweist, nämlich sowohl was den Einsatzort als auch die Einsatzzeit betrifft. Der erfindungsgemäße Photobioreaktor ist unmittelbar (oder nach Durchführung weniger Handgriffe) transportabel und kann demnach als autarke Anlage per Straße, Schiene, Luft oder See weltweit transportiert werden.

Durch die Mobilität wird der wirtschaftliche Wert der Anlage gesteigert, rascher Wertverlust durch feste Installation an einem Standort vermieden und die Einsatzmöglichkeiten des Reaktors erweitert. Des Weiteren können verschiedene Kultivationsstandorte - unter sonst exakt gleichen Bedingungen - miteinander verglichen werden.

Als autarkes und insbesondere transportables Gesamtsystem können so von Investoren Mietanlagen bereitgestellt werden, welche dem interessierten Anwender schnell zur Verfügung gestellt werden können.

Der Anwender kann so, ohne eine Fehlinvestition befürchten zu müssen, die Erzeugung von Biomasse zu dem von ihm gewünschten Zweck und an dem von ihm zugedachten Kultivationsstandort ausprobieren, oder kann eine solche Kultivation auch über einen längeren Zeitraum (Miete oder Leasing der Anlage) betreiben.

Im Ergebnis ist in erfindungsgemäßer Weise im Gegensatz zu den bisher bekannten, fest installierten Anlagen, ein völlig unabhängiges und ggf. mobiles System geschaffen worden.

Diese erfindungsgemäße, autarke Gesamtanlage lässt sich bspw. zu folgenden Verwendungen transportieren und vor Ort einsetzen:
- an mit fossilen Brennstoffen betriebenen Kraftwerken oder Zementwerken zur Nutzung bzw. Entfernung des Kohlendioxids aus dem Abgas (z.B. zur Einsparung von CO₂-Emissionszertifikaten),
- Nutzung bzw. Entfernung des CO₂ aus dem Abgas der den Photobioreaktor transportierenden Vorrichtung (bspw. LKW, Schiff),
- zur Wasser- bzw. Abwasserreinigung,
- zur Entsalzung von Wasser vor Ort,
- zur autarken Produktion von Mikroalgen an allen Standorten, und vieles mehr.

In einer bevorzugten Weiterbildung des erfindungsgemäßen Photobioreaktors weist das Gesamtsystem sämtliche erforderlichen Nebenaggregate und/oder Vorratsbehälter für zu- oder abzuführende Medien auf. Damit ist die erfindungsgemäße Vorrichtung völlig unabhängig betreibbar und kann auch an abgelegenen Standorten zur Kultivation von Biomasse verwendet werden. So können u.a. CO₂-Flaschen, Wasser- und Abwassertanks, Tanks für Nährstoffe, Tanks für Reinigungsmittel, und andere Komponenten im Gesamtsystem integriert sein.

Wie bereits erwähnt, ist im Rahmen der Erfindung ein Photobioreaktor, der als autarkes Gesamtsystem angelegt ist, und besonders bevorzugt, der transportabel ausgestattet ist.

Dazu kann das Gesamtsystem in einem Container, insbesondere in einem 20-Fuß- oder 40-Fuß-Standardcontainer nach ISO 668, oder in einem die entsprechenden Maße aufweisenden und entsprechend stapelbaren Rahmen angeordnet sein. Dabei können bauartzugelassene 20- oder 40-Fuß-Container verwendet werden, deren Stirnseite öffenbar ist. Alternativ sind Container bekannt, deren beide Vorderseiten zu öffnen sind, oder deren Längsseite sich öffnen lässt, ggf. wobei das Dach nur mit einer Plane verdeckt ist. Alternativ kann der erfindungsgemäße Photobioreaktor hinsichtlich einer optimalen Autarkie und Transportabilität in einem PKW- oder LKW-Anhänger, auf einer LKW-Wechselbrücke, in einem Güterwagon, auf einem Floß oder einem Schiff anordenbar sein.

Alternativ zu einem Container kann ein den standardisierten Containermaßen entsprechender Rahmen verwendet werden, dessen Seiten mit Steg-Doppelplatten oder auf andere geeignete Weise lichtdurchlässig ausgestaltet werden. Geeignete Rahmen sind für sich gesehen bekannt, um bspw. Flüssigkeitstanks aufzunehmen und gemäß dem Containerstandard zu lagern und/oder zu transportieren.

In einer zweckmäßigen Ausgestaltung weist der Container, der Rahmen oder dgl. Aufnahmeeinrichtung ein lichtdurchlässiges Dach und/oder eine auf- und zufahrbare Beschattungseinrichtung für das Dach (Energieschirm) und/oder Licht reflektierende Wände und/oder einen Licht reflektierenden Boden auf. Ein lichtdurchlässiges Dach ermöglicht sogar die Kultivation von Biomasse während des Transports der Vorrichtung, was einen unschätzbaren Vorteil darstellt, da ein Absterben der Kultur während des Transports aufgrund mangelnden Lichteinfalls verhindert wird.

Ein geeigneter Energieschirm kann horizontal gelagerte Vorhänge aufweisen, die durch Drähte geführt auf- und zufahrbar sind. So kann entweder in kalten Nächten die Abstrahlung von Wärme, auch von Heizungswärme, vermindert werden, während an heißen Tagen die Sonneneinstrahlung abschirmbar ist. Letzterer Aspekt ist für die Algenkultivation besonders wichtig. Bei Suspensionstemperaturen von mehr als 40° C sterben die Algen durch eine Denaturierung des Eiweißes ab. Da Photobioreaktoren aber darauf ausgelegt sind, eine möglichst große photoaktive Oberfläche aufzuweisen, besteht stets auch die Gefahr einer zu starken Aufheizung der Suspension durch die Sonneneinstrahlung. Dies kann durch die vorgeschlagene Möglichkeit einer teilweisen oder vollständigen Beschattung verhindert werden. Das Problem einer notwendigen Verminderung, der Sonneneinstrahlung besteht insbesondere auch beim Anfahren der Anlage, da bei geringen Trockenmassenanteilen das Chlorophyll in der Biomasse durch zu starke Sonneneinstrahlung zerstört werden kann.

Zur besseren Lichtausbeute können die nicht zu öffnenden und/oder die nicht lichtdurchlässigen Innenflächen des Containers oder der Aufnahmeeinrichtung reflektierend ausgestaltet sein, nämlich bspw. durch einen entsprechenden Anstrich oder eine Beschichtung. So ist eine möglichst umfassende Einstrahlung im Photosyntheseteil erreichbar.

Dem Photobioreaktor kann eine Beleuchtungseinrichtung und/oder eine Heizung und/oder eine Kühleinrichtung zugeordnet sein.

Mit einer geeigneten Beleuchtungseinrichtung ist es möglich, auch den abgeschlossenen Photobioreaktor (bspw. in einem Container) unter künstliche Licht ganzjährig zu betreiben. Dabei hat sich als besonders vorteilhaft erwiesen, die Lampen der Beleuchtungseinrichtung nicht über der Anlage anzubringen, sondern diese innerhalb der Anlage zu verteilen (die Lampen hängen bspw. in die Anlage hinein). Somit können sämtliche Bereiche des Photosyntheseteils effektiv und in allen Richtungen bestrahlt werden. Bei der herkömmlichen, von oben ausgehenden Beleuchtung ist festgestellt worden, dass die Lichtstärke an den oberen Rohren des Photosyntheseteils zu hoch, an den unteren Rohren dagegen zu gering war.

Des Weiteren hat sich als vorteilhaft erwiesen, die Vorschaltgeräte zu den einzelnen Lampen nicht in die Lampen zu integrieren, sondern in separaten Schaltkästen anzuordnen, welche neben der Anlage auf dem Boden aufgestellt werden können und somit eine geringere Beschattung verursachen.

An den Leuchtmitteln können darüber hinaus Reflektoren angebracht sein, welche eine direkte Bestrahlung der Rohre verhindern. Mit den genannten Maßnahmen konnte die Steigerung der Effizienz der Umwandlung von Strom in Biomasse von ca. 30 % gegenüber einer herkömmlichen Beleuchtung erreicht werden.

Des Weiteren konnte im Rahmen der Erfindung festgestellt werden, dass der Kühlung der Anlage - entgegen der bisherigen Meinung - eine größere Bedeutung zukommt als der Heizung. Viele Mikroalgenspezies sind in ihrem Temperaturoptimum relativ variabel, d.h. es hat keine großen Auswirkungen auf die Wachstumsrate, ob eine bestimmte Spezies bei bspw. 22° C oder 26° C kultiviert wird. Wichtig ist hingegen, dass die gewählte Temperatur möglichst konstant gehalten wird, wobei eine leichte Temperaturabsenkung in der Dunkelphase (Nacht) die Mikrobiologie positiv beeinflusst.

Eine Kühlanlage kann mit sog. Tropfschläuchen bereitgestellt werden. Dabei handelt es sich um Plastikschläuche, welche in bestimmten Abständen kleine Löcher aufweisen, durch die sie Wasser tröpfchenweise abgeben können. Solche Tropfschläuche sind aus der Pflanzenbewässerung bekannt. Die Tropfschläuche werden oberhalb des Photosyntheseteils, welcher üblicherweise aus transparenten Rohren aufgebaut ist, angeordnet. Das aus den Tropfschläuchen austretende Wasser kann an den Rohren herunterrinnen und dadurch die in den Rohren fließende Suspension kühlen. Das hierzu notwendige Kühlwasser kann vorher entionisiert werden, um Kalkflecken auf den Rohren zu vermeiden.

Das schließlich vom Photosyntheseteil auf den Boden der Anlage tropfende Wasser kann wiederverwendet werden, bspw. indem dem Boden ein Gefälle gegeben wird, wodurch sich das abfließende Kühlwasser sammeln kann.

Die Beheizung des Photobioreaktors kann im Wesentlichen bereits durch die künstliche Beleuchtung erreicht werden, was eine Kombination des Heizeffekts mit dem Biomassewachstum bedeutet.

Es ist jedoch ebenfalls möglich, die Anlage (bspw. einen Container) mit einer Boden- und/oder Rohrheizung auszustatten, wobei die Heizungsrohre direkt an der Unterseite des Photosyntheseteils verlaufen können, so dass die aufsteigende Wärme den gesamten Photosyntheseteil durchströmt.

In einer weiteren bevorzugten Ausführungsform des erfindungsgemäßen Photobioreaktors ist der Photosyntheseteil federnd gelagert. Dies ist insbesondere bei Ausführungsformen zweckmäßig, bei denen der Photosyntheseteil durch Glasröhre gebildet ist. Aufgrund der Transportabilität der Anlage sind diese unweigerlich Erschütterungen ausgesetzt. Um eine Beschädigung der empfindlichen Glasteile zu verhindern, ist daher zweckmäßig, den Photosyntheseteil federnd zu lagern. Je nach Anforderungsprofil können jedoch auch andere oder sämtliche Bauteile der Anlage federnd gelagert werden, um nämlich eine Beschädigung beim Transport und beim Auf- oder Abbau insgesamt auszuschließen.

Der Photosyntheseteil kann in einzelne Module aufgeteilt sein, ggf. wobei die Module in einem Winkel von 0° bis 90° gegen die Horizontale geneigt sind. Dabei können zwei oder mehrere Photosyntheseteile vorgesehen sein. Des Weiteren ist bevorzugt, dass der Photosyntheseteil aus Glasrohren, insbesondere aus Borosilikatglas, aufgebaut ist, und/oder dass sonstige Rohrverbindungen im Reaktor aus lebensmittelechtem Kunststoff gefertigt sind.

Der Photosyntheseteil kann demnach aus mehreren Modulen bestehen, deren Zahl variieren kann. Im Rahmen der vorliegenden Erfindung hat sich als vorteilhaft erwiesen, nicht nur einen, sondern zwei Photosyntheseteile vorzusehen, welche jeweils aus bspw. fünf Modulen bestehen können. Die Röhren des Photosyntheseteils können aus Borosilikatglas gefertigt werden, welches eine geringere Längenausdehnung als Kunststoff aufweist.

Die Verwendung von zwei Photosyntheseteilen und/oder die Aufspaltung des Photosyntheseteils in einzelne Module birgt gleich mehrere Vorteile. Zum einen wird durch die Platzierung von zwei gleichartigen Photosyntheseteilen am jeweils entgegengesetzten Ende der Anlage eine vorteilhafte Gleichgewichtslage des Gesamtsystems erreicht, insbesondere falls dieses in einem Container oder einer sonstigen geeigneten Aufnahmeeinrichtung angeordnet ist.

Des Weiteren kann bspw. ein Photosyntheseteil betrieben werden, während der andere Photosyntheseteil gereinigt oder angefahren wird. Dasselbe gilt für die Aufspaltung von Photosyntheseteilen in Module. Die Module können am Ein- und Auslauf mit einem von Hand oder elektronisch regelbaren Schieberventil ausgestattet werden, so dass sie einzeln in den Kreislauf der Anlage ab- und zugeschaltet werden können. Damit kann das photoaktive Volumen schrittweise erhöht werden, bspw. in 200 Liter-Schritten von 200 auf bis zu 2.000 Liter (bei zwei Photosyntheseteilen mit jeweils fünf Modulen à 200 Liter). Diese Möglichkeit ist wichtig für das Up-Scaling der Anlage. So ist es ratsam, den Reaktor mit einer Konzentration von ca. 0,2 g Biotrockenmasse pro Liter zu starten. Steht aber für das gesamte Anlagenvolumen nicht genügend Biomasse zur Verfügung, kann der Reaktor auch modulweise hochgefahren werden. Ohne die Aufteilung in Module wäre ein Anfahren sonst nicht möglich.

Des Weiteren ist beobachtet worden, dass die Algen keinen Schaden nehmen, wenn sie nicht ständig umgewälzt werden. Durch das zeitweise Absperren einzelner Module lässt sich so die Pumpleistung und damit auch der Stromverbrauch der Anlage reduzieren. Des Weiteren wird der Scherstress für die Algen reduziert.

Darüber hinaus kann in den Modulen - auch wenn es nur eine Systempumpe vorhanden ist - je nach Ventilstellung mit verschiedenen Fließgeschwindigkeiten gearbeitet werden. Dies ist besonders wichtig, wenn sich Algen in Teilen des Photosyntheseteils an den Innenseiten der Glasrohre absetzen. In einem solchen Fall können die betroffenen Module weiter geöffnet werden als die anderen, so dass sie stärker durchströmt werden, was zu einem Ablösen der Ablagerungen führt.

Die Glasrohre der Photosyntheseteile können mit Schrumpffolien verbunden werden. Dies kann sich jedoch als nachteilig erweisen, falls die Rohre bei Reparaturen ausgetauscht werden müssen. Alternativ lassen sich demnach Klemmverschlüsse zur Verbindung der Rohre verwenden.

Wird ein Modul vom Kreislauf abgetrennt, kann nur der unten liegende Vorlauf geschlossen werden, während der oben liegende Rücklauf offen bleiben muss, da bei völligem Abschluss die Gefahr des Zerberstens der Rohre besteht, wenn nämlich das Volumen aufgrund von Temperaturänderungen schwankt oder die Algen Sauerstoff produzieren. Daher kann der Einsatz von Überdruckventilen vorgesehen werden, welche vor dem den Rücklauf abschließenden Ventil platzierbar sind. Damit kann sowohl der Vor- als auch der Rücklauf eines Moduls geschlossen gehalten werden.

Die Module bzw. Glasröhre des Photosyntheseteils-können in horizontaler Richtung verlaufen und exakt übereinander angeordnet sein. Damit wird die Grundfläche optimal ausgenutzt und es ist zwischen den Rohren Platz für Pflege- und Wartungsarbeiten vorhanden. Nachteilig ist jedoch, dass die oben liegenden Rohre die unteren Rohre beschatten. Um dies zu umgehen, können die einzelnen Module nicht lotrecht, sondern in einem Winkel zwischen 0° und 90° gegen die Horizontale geneigt sein. Durch die Schrägstellung der Module wird eine Verschattung durch darüberliegende Rohre ganz oder teilweise verhindert, so dass jedes Rohr der vollen Lichteinstrahlung ausgesetzt ist, womit allerdings eine verminderte Raumnutzung einhergeht.

In einer weiteren vorteilhaften Weiterbildung des Erfindungsgedankens ist der Systembehälter gekapselt und/oder weist eine Heizung und/oder eine Kühlanlage und/oder eine Beleuchtung auf.

Der Systembehälter ist mit dem Photosyntheseteil verbunden und bildet den Ausgangs- und Endpunkt des Kreislaufs für die Kultursuspension. Er gleicht die Volumenänderung der Suspension bei Temperaturänderungen aus und dient zur Nährstoffzuführung und zum Gasaustausch.

Der Systembehälter kann doppelwandig ausgestaltet und so indirekt beheizbar oder kühlbar sein.

Des Weiteren soll der von den Algen durch Photosynthese produzierte Sauerstoff möglichst effizient abgeschieden werden, da er sich sonst in der Suspension anreichert und ab einer bestimmten Konzentration als Zellgift wirkt. Wird im Systembehälter eine effiziente Entfernung von Sauerstoff realisiert, wird insbesondere bei schnell wachsenden Algen das Wachstum nicht durch toxische O₂-Konzentrationen gebremst.

Dabei ist auch zu beachten, dass mit steigender Rohrlänge des Photosyntheseteils auch die Aufenthaltsdauer der Kultursuspension im Photosyntheseteil ansteigt, bis diese wieder in den Systembehälter zurück gelangt und dort den gebildeten Sauerstoff abgeben kann. Ist die Rohrlänge im Photosyntheseteil besonders lang - was im Sinne einer effektiven Kultivation bevorzugt ist - können jedoch rasch toxische Sauerstoffwerte innerhalb der Kultursuspension erreicht werden.

Eine effektive Abtrennung von Sauerstoff im Systembehälter lässt sich erreichen, indem das von oben in den Systembehälter führende Rücklaufrohr mit einem T-Stück endet, auf eine Prallscheibe stößt, oder die Suspension über einen Sprühkopf in den Behälter verteilt wird.

Durch das Versprühen der Suspension kann jedoch Schaum entstehen, der dann wieder von der Pumpe angesaugt und in die Glasröhren gedrückt werden kann. Diesbezüglich kann eine horizontale Trennwand im Systembehälter angebracht werden, die die aus dem Rücklauf strömende Suspension beruhigt.

Alternativ oder zusätzlich kann ein zweiter Systembehälter dem ersten Systembehälter nachgeschaltet werden, aus dem die dann beruhigte Suspension in den Kreislauf rückförderbar ist.

Dadurch kann ebenfalls verhindert werden, dass das zwischen Systembehälter und Systempumpe eingespeiste Kohlendioxid durch den Systembehälter zurück entweicht, anstatt - nach der Durchmischung durch die Systempumpe - im Photosyntheseteil zur Verfügung zu stehen.

Der Austrag von Sauerstoff im Systembehälter lässt sich weiter verbessern, wenn Luft in die Suspension im Behälter eingeleitet wird. Dazu kann unterhalb der Flüssigkeitsoberfläche eine balgartige Vorrichtung vorgesehen sein, die über membranartige Öffnungen, welche sich ab einem bestimmten Innendruck im Balg öffnen, Luft in vielen kleinen Bläschen im Systembehälter verströmt. Diese balgartige Vorrichtung ist vorzugsweise am Boden des Systembehälters angebracht.

In Bezug auf das Verhältnis der Größe des Systembehälters zur Gesamtanlage hat sich ein Volumenverhältnis von Photosyntheseteil und Systembehälter von 2-4 : 1 als ideal hinsichtlich der Minimierung der Schaumbildung und hinsichtlich einer Maximierung des Sauerstoffaustrags herausgestellt.

Im Hinblick auf eine effektive Verhinderung von Verunreinigungen und Beschädigungen der Anlage wird vorgeschlagen, zwischen dem Ausgang des Systembehälters und der Systempumpe ein Ventil, ein Sieb und danach ein zweites Ventil zu platzieren. Der oben zu öffnende Systembehälter ist die einzige Stelle der Anlage, an der fremde Gegenstände in die Anlage gelangen können. So können bspw. bei Arbeiten am Behälter oder beim Nachdüngen Fremdkörper in den Behälter fallen, von der Pumpe angesaugt und ungewollt in die Anlage gefördert werden. Das Sieb, dessen jeweilige Maschenweite auf die Zellgröße der zu kultivierenden Algen abgestimmt werden kann, verhindert, dass in den Betriebsbehälter gefallene Gegenstände in den Photosyntheseteil gelangen können. Durch die Absperrventile vor und nach dem Sieb kann dieses gereinigt werden, ohne die Suspension aus der Anlage ablassen zu müssen.

Um die Dunkelphase für die Kultursuspension zu minimieren, kann der Systembehälter innen beleuchtbar sein. Dies kann entweder von oben, bspw. durch an der Deckelunterseite angebrachte Lampen/LEDs realisiert werden, oder die Lampen können in transparenten Glas- oder Plastikröhren untergebracht werden, die vertikal vom Behälterdeckel bis etwa zur Behältermitte ragen.

Bei herkömmlicher Bauart ist der Deckel des Systembehälters luftdurchlässig konstruiert, um den Austrag von Sauerstoff zu gewährleisten. Dies stellt jedoch ein Kontaminationsproblem dar, da durch den Systembehälter unerwünschte Mikroorganismen in das System gelangen und sich dort stark vermehren können. Insbesondere bei der Produktion von hochreinen Algenkulturen, welche oft eine geringe Wachstumsrate aufweisen, ist eine Kontamination mit Fremdkulturen unbedingt zu vermeiden. Daher wird vorgeschlagen, den Systembehälter gekapselt auszuführen.

Dabei kann der Deckel des Systembehälters mit Schnellspannverschlüssen fixiert und dadurch zu Reinigungszwecken leicht geöffnet werden. Der produzierte Sauerstoff kann durch ein Rohr entweichen, dessen Ende - in der Art eines Siphons - mit Wasser gefüllt ist. Das Ende des Rohres kann zusätzlich mit einem Luftfilter versehen sein, so dass keine ungereinigte Umgebungsluft in den Behälter gelangen kann.

Das Eindringen von Fremdalgen und ähnlichem kann des Weiteren dadurch verhindert werden, dass im Kopf des Systembehälters ein ständiger Überdruck eingestellt wird ("Kopfspülung"). Dieser Überdruck ist dadurch erzeugbar, dass eine elektrische Pumpe gefilterte Umgebungsluft ansaugt und in den Behälter drückt. Um den Sauerstoffaustrag aus der Suspension weiter zu erhöhen, kann diese Luft durch ein vertikales Tauchrohr, an dessen Ende ein Sprühkopf angebracht ist, direkt in die Suspension gepumpt werden.

Eine weitere zweckmäßige Ausführungsform des erfindungsgemäßen Photobioreaktors zeichnet sich dadurch aus, dass zwei oder mehrere Systempumpen vorgesehen sind.

So kann bspw. ein Teil der Anlage abgeerntet und gereinigt werden, während die Kultivation im anderen Teil weiterläuft. Dies ist besonders wichtig, weil das Up-Scaling der für den Photobioreaktors benötigten Starterkultur aus dem Labormaßstab bis zu mehreren Monaten dauern kann. Ist der Reaktor jedoch im hier beschriebenen Sinne teilbar, können beide Hälften, und zwar im laufenden Betrieb, nacheinander gereinigt werden, während in der jeweils anderen Hälfte die Kultur gehalten wird. Dadurch erübrigt sich das wiederholte Anfahren des gesamten Reaktors und die lange Wartezeit, bis die neu angesetzte Kultursuspension den gewünschten Feststoffgehalt an Biomasse aufweist.

Des Weiteren ist in Bezug auf diese Ausführungsform zu beachten, dass manche Algen hochwertige Inhaltsstoffe nur unter bestimmten Stressbedingungen bilden. So ist z.B. bei der Produktion von Astaxanthin aus Haematococcus pluvialis (Blutregenalge) eine vegetative, "grüne Phase", und eine Stressphase ("Rotphase") notwendig: In der Grünphase wächst die Alge normal, in der Rotphase wird sie gestresst und bildet dadurch Astaxanthin. Für einen solchen zweiphasigen Produktionsprozess sind - sofern man kontinuierlich und nicht im Batch-System arbeiten will - ansonsten zwei Reaktoren notwendig. Bei einem wie hier vorgeschlagenen teilbaren Reaktor jedoch kann zunächst der ganze Reaktor "grün" gefahren werden, dann wird ein Teil abgetrennt und unter Stress die Rotphase initiiert und abgeerntet, so dass dann wieder aus dem "grünen" Teil der Suspension Algenmaterial für die Rotphase in der anderen Anlagenhälfte zur Verfügung steht.

Hinsichtlich einer umfassenden Regelbarkeit des Kultivationsvorgangs wird eine Ausgestaltung des erfindungsgemäßen Photobioreaktors vorgeschlagen, bei der mittels der Regelungseinheit der Lichteintrag und/oder ein Heizen und/oder ein Kühlen des Reaktors, insbesondere abhängig von der Suspensionstemperatur, ermöglicht ist.

Zunächst sollten die nachfolgend aufgezählten wichtigen Parameter für den Kultivationsvorgang durch die Regelungseinheit erfassbar und überwachbar sein. Der Druck in der Anlage sollte konstant überwacht werden. Bei Überschreitung des Sollwertes, der durch die Belastbarkeit der verwendeten Bauteile, insbesondere des Photosyntheseteils bestimmt wird (insbesondere bei einem Überdruck von ca. 0,8 bar), wird die Systempumpe automatisch herabgeregelt, um eine Beschädigung der Anlage, insbesondere ein Zerbersten der Rohre zu verhindern. Des Weiteren kann die Temperatur der Suspension an einem oder mehreren Messpunkten aufnehmbar sein. Mittels einer Messung der optischen Dichte kann ein ungefährer Anhaltspunkt über das Algenwachstum erhalten werden, für die genaue Ermittlung der Wachstumsdaten kann jedoch im Labor der Trockenmassegehalt bestimmt werden.

Über die Drehzahl der Systempumpe, welche über einen Frequenzumrichter steuerbar ist, kann die Fließgeschwindigkeit der Kultursuspension und der Anlagendruck eingestellt werden.

Schließlich kann die Kohlendioxid-Einspeisung in Abhängigkeit vom pH-Wert der Kultursuspension geregelt werden. Mit ansteigender Kohlendioxid-Aufnahme durch die Algen steigt der pH-Wert an. Bei einem einstellbaren oberen Schwellenwert kann das Magnetventil der Kohlendioxid-Einspeisung geöffnet werden, welches sich vor der Pumpe befindet. Das eingespeiste Kohlendioxid wird durch die Pumpe verwirbelt. Daraufhin sinkt der pH-Wert, bis an einem unteren Schwellenwert die Zuspeisung von Kohlendioxid wieder gestoppt wird.

Des Weiteren wird vorgeschlagen, dass mittels der Regelungseinheit der Lichteintrag, die Belüftung und/oder die Beschattung und/oder die Heizung und/oder die Kühlung der Anlage in Abhängigkeit von der Suspensionstemperatur regelbar ist. Über die Aufzeichnung aller relevante Umwelt- und Reaktordaten können statistische Auswertungen (z.B. Wachstumsrate in Bezug auf die Lichteinstrahlung oder die Temperatur) vorgenommen werden.

In einer weiteren Weiterentwicklung der Regelungseinheit können für jeden Parameter Ober- und Untergrenzen definiert sein, deren Über- bzw. Unterschreitung eine Alarmmeldung, bspw. auf ein Handy, auslösen kann.

Es wird vorgeschlagen, die Messsonden für die Suspensionstemperatur, den pH-Wert und die optische Dichte nicht im Betriebsbehälter, sondern in einer Bypass-Leitung zwischen Vor- oder Rücklauf und Betriebsbehälter anzuordnen. Anfang und Ende der Bypass-Leitung können mit Kugelhähnen verschließbar sein, so können die Sonden problemlos im laufenden Betrieb entnommen werden. Des Weiteren sollte vor der Bypass-Leitung ein Siebfilter angebracht sein, um ggf. in der Suspension befindliches Reinigungsgranulat (siehe unten) abzuhalten, da dieses sonst die Sonden beschädigen könnte.

Am Systembehälter, dem Vor- oder Rücklauf oder in einer Bypassleitung kann ein Probeentnahmehahn vorgesehen werden, an dem zu Analysezwecken Proben entnommen werden können, ohne das System zu öffnen. Dabei ist wichtig, dass der Hahn nach unten gerichtet ist und automatisch leer läuft, so dass keine zersetzten oder sich zersetzenden Produktreste in die Anlage zurück gelangen können.

Schließlich kann die Ernteeinrichtung eine Zentrifuge oder einen Sedimentationsbehälter aufweisen. Ein Sedimentationsbehälter ist hierbei preisgünstiger und schonender für das abgetrennte Material, kann jedoch nur bei Algenspezies mit einer Zellgröße ab ca. 50 µm eingesetzt werden, und arbeitet nicht so effizient wie eine Zentrifuge. Die Ernteeinrichtung ist mit einer Entnahme- oder Fördermöglichkeit für die geerntete Biomasse vorzusehen. Der Klarlauf (= Kulturmedium ohne Mikroalgen) wird in den Kreislauf zurückgeführt, kann jedoch nach Wahl auch verworfen werden, um durch frisches Medium (Wasser und darin angelöste Stoffe) ersetzt zu werden (siehe unten).

Der erfindungsgemäße Photobioreaktor kann im Rahmen weiterer vorteilhafter Ausgestaltungen durch periphere Anlagen ergänzt werden, welche bei Bedarf zugeschaltet werden können. Dabei kann ggf. ein Bypass im Kultivationskreislauf vorgesehen werden, in den die periphere oder externe Anlage zuschaltbar ist.

So können Nährstoffe aufgelöst, autoklaviert und über eine Dosierpumpe langsam in die Suspension eingeleitet werden. Dies bietet gegenüber der herkömmlichen Düngung, bei der Nährsalze in kristalliner Form in den Systembehälter gegeben werden, folgende Vorteile:
- der Systembehälter (und damit der gesamte Photobioreaktor) kann gekapselt werden,
- eine Fremdalgenkontamination über verunreinigte Nährstoffe wird ausgeschlossen,
- es ist sichergestellt, dass die Nährstoffe tatsächlich gelöst sind und sich nicht etwa ungelöste Salze am Boden des Systembehälters sammeln,
- eine Überkonzentration von Nährsalzen aufgrund einer nicht zeitverzögerten, chargenweisen Zuführung wird durch kontinuierliches Einspeisen verhindert, und
- durch kontinuierliches Einspeisen der notwendigen Nährstoffe über einen langen Zeitraum kann die Nährstoffkonzentration immer annäherend konstant gehalten werden, was den Stress für die Algen verringert, dem sie sonst bei diskontinuierlicher Nachdüngung zwei oder dreimal in der Woche ausgesetzt wären.

Weiterhin kann zur Entfernung von Kontaminationen ein Spinfilter in einer Bypass-Leitung vorgesehen werden, welcher kontinuierlich Fremdalgen ausfiltert, sofern diese einen Größenunterschied zur Zielkultur aufweisen.

Schließlich kann zur Erzeugung von Stress (z.B. zur Astaxanthin-Bildung) oder auch zur Bekämpfung von Kontaminanten die Suspension mittels einer Bypass-Leitung durch das Licht einer UV-Lampe geführt werden.

Die eingangs genannte Aufgabe ist in Bezug auf ein Verfahren zur Kultivation von Biomasse mittels Photosynthese mit den Merkmalen des Patentanspruchs 15 gelöst. Danach ist ein eingangs genanntes Verfahren durch die Verwendung eines erfindungsgemäßen Photobioreaktors ausgestaltet und weitergebildet.

Erfindungsgemäße Vorteile sowie vorteilhafte Ausgestaltungen und Weiterbildungen des Verfahrens ergeben sich aus den vorstehenden Ausführungen bezüglich der Vorrichtung, welche das Verfahren jedoch ausdrücklich einschießen.

Weitere vorteilhafte Ausgestaltungen des erfindungsgemäßen Verfahrens sind den Patentansprüchen 16 bis 18 entnehmbar.

Im Rahmen der Erfindung ist festgestellt worden, dass mit dem erfindungsgemäßen Photobioreaktor sowie unter Anwendung des erfindungsgemäßen Verfahrens überraschend lange Kultivationslaufzeiten, demnach eine langzeit-stabile Produktion erreichbar ist. So ist bisher davon ausgegangen worden, dass ein Photobioreaktor oft schon nach wenigen Wochen, jedenfalls aber nach einigen Monaten abgefahren und gereinigt werden müsse, weil spätestens dann immobilisierende Algen eine toxische Nitritbelastung bzw. eine ungünstige Mikrobiologie hervorrufen würden.

Tatsächlich konnte im Rahmen der Erfindung erreicht werden, einen Algenstamm über ein Jahr ohne Reinigung zu kultivieren.

Dies ist zum Einen durch geringe Schwankungen der Strömungsgeschwindigkeit in der Anlage erreichbar. Algen immobilisieren nur an den Stellen, an denen die Strömungsgeschwindigkeit absinkt. Aufgrund des vorteilhaften Designs der Anlage verbunden mit einer entsprechenden Stromführung und Regelung kann jedoch an jeder Stelle der Anlage eine annäherend konstante Strömungsgeschwindigkeit erreicht werden, so dass Immobilisierungen minimiert werden.

Des Weiteren wird vorgeschlagen, im Hinblick auf eine lange Kultivationslaufzeit der Kultursuspension die Innenseiten der die Kultursuspension beinhaltenden Teile des Bioreaktors, insbesondere den Photosyntheseteil, und insbesondere Glasrohre des Phototsyntheseteils, periodisch oder permanent durch die Mitförderung von Reinigungskörpern, insbesondere von Reinigungsgranulat, abzureinigen. Das Reinigungsgranulat kann in die Suspension eingebracht werden und durch die Mitförderung im Strom etwaige Immobilisierungen an den Innenseiten der Rohre von innen abschleifen. Als besonders geeignet hat sich die Verwendung von Kunststoffgranulaten erwiesen.

Besonders bevorzugt ist, zumindest zwei Sorten von Reinigungsgranulaten gleichzeitig zu verwenden. Eine Sorte kann dabei eine etwas geringere Dichte als die Suspension aufweisen, während die zweite Sorte eine höhere Dichte aufweiset. So lassen sich sowohl die Ober- als auch die Unterseiten der Glasrohre abreinigen. Das Reinigungsergebnis kann durch die Verwendung einer dritten Sorte, welche möglichst dieselbe Dichte wie die Suspension aufweist, noch weiter verbessert werden. So lassen sich neben den Ober- und Unterseiten der Rohre auch deren Seitenbereiche effektiv reinigen.

Als besonders effektiv hat sich hierbei eine Konzentration der verschiedenen Reinigungsgranulatsorten von jeweils ca. 3,5 kg pro m³ Kultursuspension erwiesen. Durch den Einsatz von Reinigungsgranulaten kann auch die Drehzahl der Systempumpe reduziert werden, was sowohl den Stromverbrauch und damit auch einen erheblichen Teil der Betriebskosten, als auch den Scherstress für die Algen minimiert.

Durch den periodischen oder permanenten Einsatz von Reinigungsgranulaten kann ein Auseinanderbau der Anlage und ein mechanisches Reinigen der Glasrohre vermieden werden. Dadurch lassen sich die Betriebskosten stark verringern, wird die Standzeit der Anlage erhöht und wird nicht zuletzt die Gefahr der mechanischen Beschädigung der Anlage durch den Reinigungsvorgang verringert. Sofern Reinigungsgranulate zur Reinigung der Rohrinnenseiten eingesetzt werden, bietet es sich an, etwaig vorhandene Sensoren in mit Sieben geschützten Bypassleitungen anzuordnen, wo sie von dem Reinigungsgranulat unversehrt bleiben.

Des Weiteren wird im Hinblick auf die Einsparung von Betriebskosten bei gleichzeitig guten Kultivationsergebnissen vorgeschlagen, den Kultivationsschritt einem Regime künstlicher Beleuchtung zu unterwerfen, wobei in Bezug auf den Tagesablauf eine Verlängerung des Tageslichts mit anschließender Dunkelphase (Nacht) und/oder in Bezug auf die Jahreszeiten ein kurzer Winter simuliert wird.

Im Rahmen der vorliegenden Erfindung hat sich eine Beleuchtungsintensität von 75 W/m² als vorteilhaft herausgestellt. Des Weiteren wurde überraschenderweise festgestellt, dass eine Beleuchtung über die gesamte Nachtzeit nicht mehr Biomassewachstum bringt, als wenn alternativ ein Belichtungsblock am Abend und am Morgen eingestellt wird, also einfach eine Verlängerung des Tageslichts simuliert wird. Auf die kostenintensive Nachtbeleuchtung kann daher ggf. verzichtet werden.

Des Weiteren hat sich gezeigt, dass eine Unterbrechung der Beleuchtung für ca. 1-2 Stunden den Biomassezuwachs kaum beeinträchtigt, so dass im Sinne einer Energieeinsparung öfters solche Beleuchtungspausen vorgesehen werden können.

Zusätzlich hat sich gezeigt, dass sich während der Dunkelphase die Drehzahl der Systempumpe ohne nachteilige Auswirkungen um ca. 15 % bis ca. 25 % reduzieren lässt. Dadurch werden die Energiekosten gesenkt und der Scherstress für die Algen in dieser Zeit vermindert.

Schließlich hat sich erwiesen, dass die Algen je nach natürlicher Strahlungsmenge unterschiedlich auf die künstliche Beleuchtung reagieren. So ist kaum zusätzliches Biomassewachstum durch künstliche Beleuchtung erreicht worden, falls nach einem Tag mit langer und starker Sonneneinstrahlung (Sommer), welcher bereits ein starkes Biomassewachstum gebracht hat, noch belichtet worden ist. Auf der anderen Seite ist gefunden worden, dass im Winter, wenn sich die Algen sowieso an ein geringes Strahlungsniveau adaptiert haben, eine wesentlich weniger intensive künstliche Beleuchtung ausreicht, um das gesamte Wachstumspotential der Biomasse auszuschöpfen.

In besonders überraschender Weise ist im Rahmen der Erfindung festgestellt worden, dass die Algen ein jahreszeitenabhängiges Wachstumspotential aufweisen, und sozusagen einen "inneren Kalender" zu besitzen scheinen. So wurde festgestellt, dass die Wachstumsrate im Herbst geringer sind als in einem hinsichtlich der Temperatur und der Sonnenstundenzahl vergleichbaren Frühjahreszeitraum. Ausgehend von dieser Erkenntnis scheint es vorteilhaft, durch Beschattung und anschließende künstliche Beleuchtung einen kurzen Winter zu simulieren und so mehrere starke Wachstumsperioden pro Jahr zu erzielen, demnach den Jahreszeitablauf in optimaler Weise zu simulieren.

Schließlich wird im Hinblick auf ein wirtschaftlich optimiertes Kultivationsergebnis eine Ausgestaltung des Verfahrens vorgeschlagen, bei der der Kultivationszyklus einem Ernteregime unterworfen wird, wonach ca. alle 14 Tage ca. ein Drittel des Volumens der Kultursuspension erneuert werden, insbesondere dadurch, dass der aus der Ernteeinrichtung zurückgehaltene Klarlauf verworfen und durch frisches Kultivationsmedium ersetzt wird.

Dabei ist zu beachten, dass das Kultivationsziel in der Regel die Maximierung des Biomassewachstums ist, wobei bei wenigen Spezies auch die Maximierung des Wirkstoffgehalts je Zeiteinheit in den Vordergrund gestellt wird.

Mikroalgen vermehren sich durch Zellteilung, so dass eine Wachstumskurve entsteht, die zunächst nur langsam ansteigt, dann in einen exponentiellen Zuwachs übergeht und schließlich wieder abflacht, nämlich wenn die Dichte so hoch ist, dass sich die Zellen gegenseitig beschatten oder - bei einigen Arten - wachstumshemmende Stoffe ausgeschüttet werden.

Es hat sich als vorteilhaft erwiesen, die Wachstumskurve möglichst in jenem Bereich zu belassen, an dem sie den stärksten Anstieg aufweist. Beginnt sie abzuflachen, wird das Ernteregime eingeleitet. Dazu wird ein Teil der Suspension mittels eines Bypasses über die Ernteeinrichtung geleitet. Dabei handelt es sich um einen Separator (Zentrifuge) oder - bei großzelligen Algen - auch um einen einfachen Sedimentationsbehälter. Dort werden die Algen sedimentiert und abgeschieden, der weiterhin Nährstoffe enthaltende Klarlauf kann (ggf. nach einer Aufreinigung) wieder in die Anlage zurückgeführt werden, wodurch der Bedarf an Wasser und Nährsalzen verringert wird. Dieses Ernteregime stellt die wirtschaftlichste Kultivationsform dar.

Allerdings ist im Rahmen der vorliegenden Erfindung festgestellt worden, dass die Algen auf lange Sicht Stoffe in die Suspension absondern, die nicht vom Separator abgeschieden werden können. Diese Stoffe führen ab einer bestimmten Konzentration dazu, dass der - je nach verwendeter Separatortechnik - bei der Separation zu erzielenden Trockenmasseanteil von ca. 15-20 % auf nur noch ca. 4-6 % sinkt, was die weitere Verarbeitung des Ernteguts unwirtschaftlich macht.

Daher hat sich als vorteilhaft erwiesen, alle zwei Wochen ca. ein Drittel des Volumens auszutauschen, d.h. den Klarlauf zu verwerfen und frisches Kultivationsmedium aufzufüllen.

Im Hinblick auf dieses Verwerfen des Klarlaufs wie auch insgesamt im Hinblick auf die Entsorgung von Abwasser aus der Anlage wird vorgeschlagen, dieses zunächst in einem Tank zu sammeln und dort bspw. mit ca. 4 % Wasserstoffperoxyd (H₂O₂) oder auch mit UV-Licht zu behandeln, so dass evtl. noch vorhandene Algen oder sonstige Mikroben abgetötet werden. Damit können keine lebenden Organismen aus dem System in die Umgebung entweichen.

Es gibt nun verschiedene Möglichkeiten, die Lehre der vorliegenden Erfindung in vorteilhafter Weise auszugestalten und weiterzubilden. Dazu ist einerseits auf die nachgeordneten Patentansprüche und andererseits auf die nachfolgende Erläuterung zweier bevorzugter Ausführungsbeispiele des erfindungsgemäßen Photobioreaktors anhand der Zeichnung zu verweisen. In Verbindung mit der Erläuterung der bevorzugten Ausführungsbeispiele des Photobioreaktors anhand der Zeichnung werden auch im Allgemeinen bevorzugte Ausgestaltungen und Weiterbildungen der Lehre, unter Einschluss des erfindungsgemäßen Verfahrens, erläutert. In der Zeichnung zeigen
- Fig. 1: eine teilweise geschnittene, perspektivische Ansicht eines ersten Ausführungsbeispiels des erfindungsgemäßen Photobioreaktors,
- Fig. 2: eine perspektivische Ansicht eines zweiten Ausführungsbeispiels des erfindungsgemäßen Photobioreaktors,
- Fig. 3: eine Seitenansicht beider Ausführungsbeispiele, und
- Fig. 4: eine von oben gesehene Darstellung beider Ausführungsbeispiele.

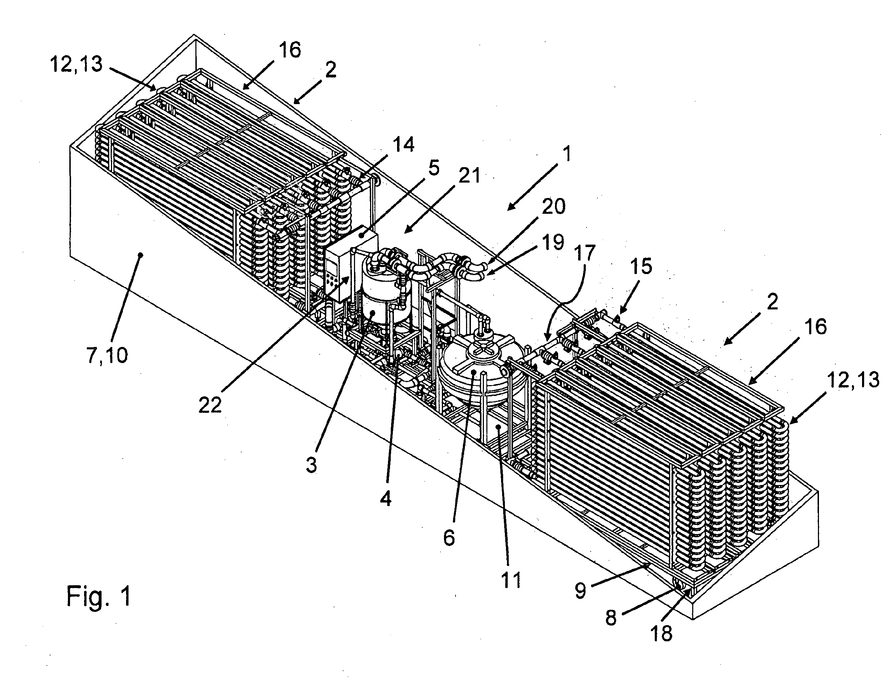

Fig. 1 zeigt eine teilweise geschnittene, perspektivische Ansicht eines ersten Ausführungsbeispiels des erfindungsgemäßen Photobioreaktors. Der Photobioreaktor ist dabei in erfindungsgemäßer Weise als autarkes und transportables Gesamtsystem 1 ausgelegt.

Im Einzelnen weist das Gesamtsystem 1, d.h. der erfindungsgemäße Photobioreaktor, zwei Photosyntheseteile 2 auf, welche jeweils am Ende der Vorrichtung angeordnet sind. In der Mitte der Vorrichtung sind ein Systembehälter 3, eine Systempumpe 4, eine Regelungseinheit 5 und eine Ernteeinrichtung 6 angeordnet.

In erfindungsgemäßer Weise ist das Gesamtsystem 1 autark und transportabel ausgelegt. Dazu ist die Anlage beim vorliegenden Ausführungsbeispiel in einem Container 7, nämlich in einem Standardcontainer nach ISO 668, angeordnet. Sowohl die beiden Photosyntheseteile 2 als auch die mittlere Baugruppe des Gesamtsystems 1 sind des Weiteren jeweils auf Rollen 8 fahrbaren Gestellen 9 zugeordnet, um einen schnellen Be- und Entladevorgang des Containers 7 zu gewährleisten.

Der Container 7 verfügt über ein transparentes, lichtdurchlässiges Dach (nicht dargestellt) sowie über Licht reflektierende Wände 10 sowie einen Licht reflektierenden Boden 11. Damit ist eine optimale Nutzung der eingestrahlten Sonnenenergie und/oder des eingestrahlten künstlichen Lichts gewährleistet.

Der Container 7 verfügt des Weiteren über eine automatische Beschattungseinrichtung für das Dach (Energieschirm), welcher hier nicht dargestellt ist. Daneben verfügen beide Photosyntheseteile 2 über eine künstliche Assimilationsbeleuchtung, welche in dieser Darstellung nicht zu sehen ist. Zusätzlich kann eine Bodenheizung vorgesehen werden, welche bspw. elektrisch oder durch einen Heizkreislauf (bspw. Warmwasser) betreibbar ist.

Beide Photosyntheseteile 2 bestehen hier aus jeweils fünf einzeln zu- und abschaltbaren Modulen 12. Die Module 12 und damit die Photosyntheseteile 2 bestehen ihrerseits aus Glasrohren 13 aus Borosilikatglas, welche übereinander liegend angeordnet sind. Die Glasrohre 13 werden über Schrumpfschläuche oder bevorzugt (weil demontierbar) durch Klemmverschlüsse miteinander verbunden.

Jedes Modul 12 der Photosyntheseteile 2 ist über ein regelbares Ventil 14 einzeln ansteuerbar, so dass im vorliegenden Fall das photoaktive Volumen in 200 Liter-Schritten von 200 Liter bis auf 2.000 Liter variiert werden kann. Zur Verhinderung eines Berstens der Glasrohre 13 im Falle eines Überdruckaufbaus bei Verschluss des Ein- und Auslasses sind zusätzlich Überdruckventile 15 vorgesehen.

Über jedem Photosyntheseteil 2 ist ein Sprühsystem 16 zur Kühlung und Reinigung der Glasrohre 13 angeordnet. Das Sprühsystem 16 wird zweckmäßigerweise mit entionisiertem Wasser betrieben, so dass sich auf den Glasrohren 13 keine Kalkflecken bilden.

Zur noch verbesserten Kühlung des Gesamtsystems 1 können an einer oder beiden Stirnseiten des Containers 7 Ventilatoren angebracht werden (hier nicht dargestellt), deren Luftstrom zusätzlich durch mit Wasser getränktes Textilmaterial geführt werden kann. Die Verrohrung außerhalb der Photosyntheseteile 2 besteht aus Kunststoffrohren 17 aus lebensmittelechtem Kunststoff. Hierdurch ist eine optimale Chemikalien- und Salzresistenz gegeben.

Neben der Mobilität der einzelnen Baugruppen aufgrund der auf Rollen 8 fahrbaren Gestelle 9 umfasst jedes Gestell 9 mehrere Standfüße 18, welche gefedert und gedämpft ausgeführt sind. Dadurch werden Beschädigungen, insbesondere der Photosyntheseteile 2, während des Transports und/oder während des Auf- und Abbaus vermieden. Zum schnelleren Auf- und Abbau der Anlage 1 können die auf Gestellen 9 angeordneten Baugruppen mittels Schnellverschlüssen miteinander verbunden oder voneinander getrennt werden.

Die in der Mitte des Gesamtsystems 1 angeordnete Baugruppe umfasst insbesondere den Systembehälter 3, die Systempumpe 4, die Regelungseinheit 5 und die Ernteeinrichtung 6. Der Systembehälter 3 dient zuallererst zum Volumenausgleich für die Kultursuspension. Aus dem Systembehälter 3 wird die Kultursuspension in die Photosyntheseteile 2 geleitet. Des Weiteren erfolgt im Systembehälter 3 die Nährstoffzuführung und der Gasaustausch.

Stromabwärts des Systembehälters 3 ist die Systempumpe 4 angeordnet, wobei vor der Systempumpe 4 ein austauschbarer Filter zum Schutz der Anlage platziert ist.

Die Ernteeinrichtung 6 besteht aus einem Separator (Zentrifuge). Alternativ kann jedoch auch ein Sedimentationsbehälter vorgesehen werden. Hier wird die kultivierte Biomasse aus der Suspension, welche stets durch die genannten Bauteile im Kreislauf geführt wird, abgetrennt. Die abgetrennte Biomasse kann danach der vorgesehenen Verwertung zugeführt werden. Der Klarlauf aus der Ernteeinrichtung 6 wird in den Kreislauf zurückgeführt oder kann, ggf. periodisch, verworfen werden. Dabei ist es vorteilhaft, das Abwasser zunächst in einem Tank zu sammeln und dort mit ca. 4% Wasserstoffperoxid (H₂O₂) zu mischen, wodurch evtl. noch vorhandene Algen abgetötet werden, damit keine Algen aus dem System in die Umgebung gelangen können.

Im mittleren Bereich des Gesamtsystems 1 sind Rohre für die Wasserzufuhr 19 und den Sauerstoffaustrag 20 zu erkennen. Beide Leitungen sind dem Systembehälter 3 zugeordnet.

Links vom Systembehälter 3 ist die Regelungseinheit 5 angeordnet, welche die Betriebsparameter des Gesamtsystems 1 überwacht und sämtliche Regelungsaufgaben übernimmt.

Hinter der Regelungseinheit 5 ist ein Freiraum 21 für weitere Nebenaggregate und/oder Vorratsbehälter für zu- oder abzuführende Medien freigehalten. Hier können bspw. CO₂-Flaschen, Wasser- und Nährstofftanks oder weitere Regelungs- und Überwachungseinheiten platziert werden. So kann bspw. ein Nährstofftank mit der automatisierbaren Nährstoffdösierung 22 verbunden werden, wodurch die Gesamtanlage noch unabhängiger wird.

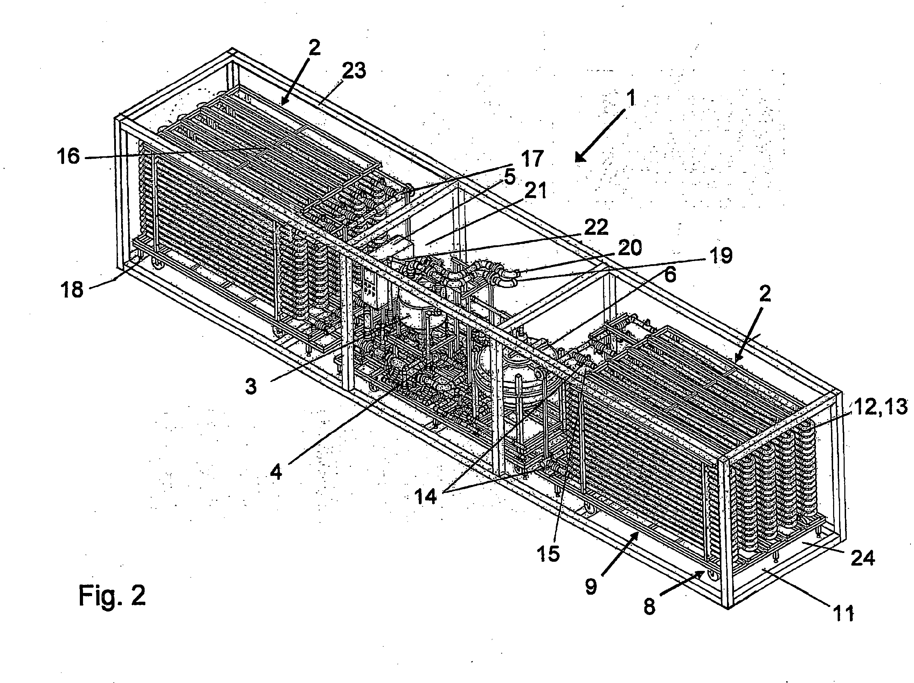

Fig. 2 zeigt eine perspektivische Ansicht eines zweiten Ausführungsbeispiels des erfindungsgemäßen Photobioreaktors. Die Baugruppen des Gesamtsystems 1 sind hierbei identisch, das Gesamtsystem 1 ist jedoch abweichend zu Fig. 1 nicht in einem Container, sondern in einem Rahmen 23 angeordnet, welcher über eine geeignete Bodenplatte 24 verfügt. Der Rahmen 23 weist die Standardmaße eines ISO-Containers auf, so dass dieser entsprechend handhabbar und transportierbar ist.

In Bezug auf die weiteren dargestellten Einzelheiten kann auf die Beschreibung zu Fig. 1 verwiesen werden.

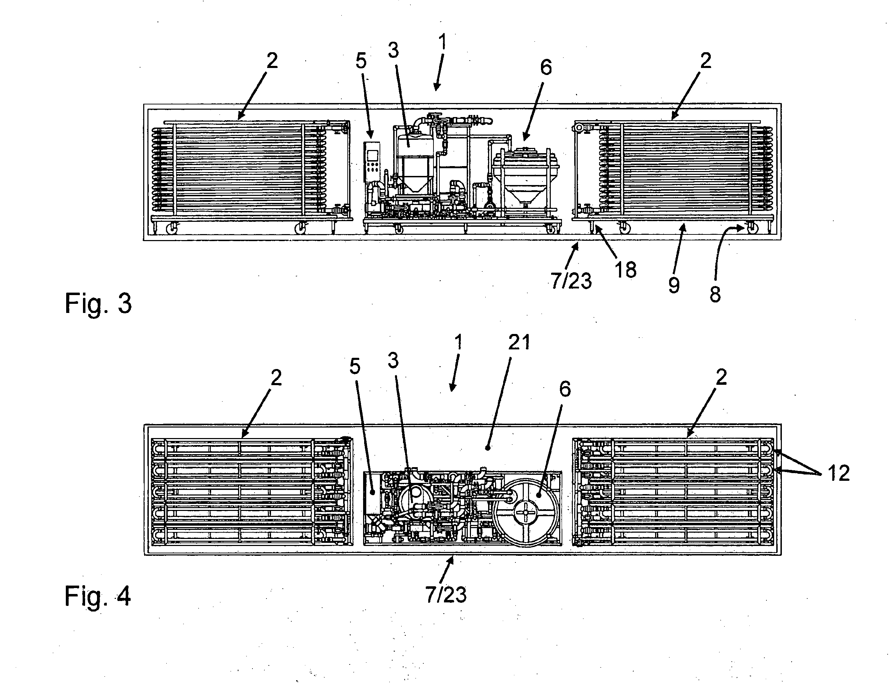

Fig. 3 zeigt die Seitenansicht beider Ausführungsformen aus Fig. 1 und Fig. 2, wobei in Bezug auf Fig. 1 hier eine geschnittene Ansicht dargestellt ist. In der Seitenansicht ist gut zu erkennen, dass das Gesamtsystem 1 aus drei Baugruppen gebildet wird, wobei auf beiden Seiten ein Photosyntheseteil 2 angeordnet ist.

Sämtliche Baugruppen sind auf Gestellen 9 angeordnet, welche über Rollen 8 verschiebbar ausgestaltet sind.

Die Baugruppen können am Einsatzort rasch entladen und mit Schnellverschlüssen wieder miteinander verbunden werden. So ist am Einsatzort (in einer Halle oder auch im Freien) ein Betrieb des Systems 1 möglich. Alternativ kann das System 1 innerhalb des Containers 7 oder des Rahmens 23 in Betrieb genommen werden.

Die Gestelle 9 sind über gefederte und gedämpfte Standfüße 18 federnd gelagert.

Fig. 4 zeigt schließlich eine von oben gesehene Ansicht der Ausführungsformen aus Fig. 1 und Fig. 2. Hier ist gut zu erkennen, dass jeder Photosyntheseteil 2 aus jeweils fünf einzeln zuschaltbaren Modulen 12 aufgebaut ist, so dass sich eine hervorragende Anpassbarkeit des Anlagevolumens an den Bedarf erzielen lässt.

Des Weiteren wird deutlich, dass auf der Rückseite der mittleren Baugruppe ein ausreichend großer Freiraum 21 zur Bevorratung weiterer Nebenaggregate und/oder Vorrats- oder Abfallbehälter vorhanden ist.

Im Ergebnis lässt sich in einem einzigen Standardcontainer sowohl das Gesamtsystem 1 des erfindungsgemäßen Photobioreaktors als auch sämtliche benötigten Aggregate und/oder Betriebsmittel vorhalten. Hierdurch ergibt sich eine praktisch weltweite Einsetzbarkeit des erfindungsgemäßen Photobioreaktors, auch in abgelegenen Gebieten. Der erfindungsgemäße Photobioreaktor ist als Gesamtsystem 1 schnell und einfach zu transportieren und ist vor Ort in kürzester Zeit einsatzbereit. Des Weiteren kann auch während des Transports der Kultivationsbetrieb aufrecht erhalten werden, was einen unschätzbaren Vorteil gegenüber bekannten Anlagen darstellt.

Abschließend sei hervorgehoben, dass die voranstehend beschriebenen Ausführungsbeispiele die beanspruchte Lehre erörtern, diese jedoch nicht auf die Ausführungsbeispiele einschränken.

### Bezugszeichenliste

- 1: Gesamtsystem
- 2: Photosyntheseteil
- 3: Systembehälter
- 4: Systempumpe
- 5: Regelungseinheit
- 6: Ernteeinrichtung
- 7: Container
- 8: Rolle
- 9: Gestell
- 10: Wand
- 11: Boden
- 12: Modul (Photosyntheseteil)
- 13: Glasrohr
- 14: Ventil
- 15: Überdruckventil
- 16: Sprühsystem
- 17: Kunststoffrohr
- 18: Standfuß
- 19: Wasserzufuhr
- 20: Sauerstoffaustrag
- 21: Freiraum
- 22: automatisierbare Nährstoffdosierung
- 23: Rahmen
- 24: Bodenplatte

## Patentansprüche

1. Photobioreaktor zur Kultivation von Biomasse mittels Photosynthese, wobei die Biomasse Algen aufweist, wobei innerhalb des Bioreaktors einer wässrigen Biomasse enthaltenden Kultursuspension Licht und ggf. Kohlendioxid und/oder Nährstoffe zuführbar sind, und wobei der Photobioreaktor zumindest die folgenden Bauteile aufweist:
- einen Photosyntheseteil (2),
- einen System- oder Ausgleichsbehälter (3),
- eine Systempumpe (4),
- eine Regelungseinheit (5), und
- eine Separations- bzw. Ernteeinrichtung (6),
wobei der Photobioreaktor als autarkes Gesamtsystem (1) ausgelegt ist,
**dadurch gekennzeichnet, dass** das Gesamtsystem (1) in einem Container (7) oder in einem die entsprechenden Maße aufweisenden und entsprechend stapelbaren Rahmen (23) angeordnet ist.

2. Photobioreaktor nach Anspruch 1, **dadurch gekennzeichnet, dass** das Gesamtsystem (1) sämtliche erforderlichen Nebenaggregate und/oder Vorratsbehälter für zu- oder abzuführende Medien umfasst.

3. Photobioreaktor nach Anspruch 1 oder 2, **dadurch gekennzeichnet, dass** das Gesamtsystem (1) transportabel ist.

4. Photobioreaktor nach einem der Ansprüche 1 bis 3, **dadurch gekennzeichnet, dass** das Gesamtsystem (1) in einem 20-Fuß- oder 40-Fuß- Standardcontainer (7) nach ISO 668, oder in einem die entsprechenden Maße aufweisenden und entsprechend stapelbaren Rahmen (23) angeordnet ist, oder dass das Gesamtsystem (1) auf einem PKW- oder LKW-Anhänger, einer LKW-Wechselbrücke, einem Güterwagon, einem Floß oder einem Schiff anordenbar ist, wobei der Container (7), der Rahmen (23) oder dergleichen Aufnahmeeinrichtung ein lichtdurchlässiges Dach und/oder eine auf- und zufahrbare Beschattungseinrichtung für das Dach (Energieschirm) und/oder Licht reflektierende Wände (10) und/oder einen Licht reflektierenden Boden (11) aufweist.

5. Photobioreaktor nach einem der Ansprüche 1 bis 4, **dadurch gekennzeichnet, dass** dem Reaktor eine Beleuchtungseinrichtung und/oder eine Heizung und/oder eine Kühleinrichtung zugeordnet ist.

6. Photobioreaktor nach einem der Ansprüche 1 bis 5, **dadurch gekennzeichnet, dass** der Photosyntheseteil (2) federnd gelagert ist und/oder dass der Photosyntheseteil (2) in einzelne Module (12) aufgeteilt ist, ggf. wobei die Module (12) in einem Winkel von 0° bis 90° gegen die Horizontale geneigt sind und/oder dass zwei oder mehrere Photosyntheseteile (2) vorgesehen sind.

7. Photobioreaktor nach einem der Ansprüche 1 bis 6, **dadurch gekennzeichnet, dass** der Photosyntheseteil (2) aus Glasrohren (13), insbesondere aus Borosilikatglas, aufgebaut ist, und/oder sonstige Rohrverbindungen (17) im Reaktor aus lebensmittelechtem Kunststoff gefertigt sind.

8. Photobioreaktor nach einem der Ansprüche 1 bis 7, **dadurch gekennzeichnet, dass** der Systembehälter (3) gekapselt ist und/oder eine Heizung und/oder eine Kühlanlage und/oder eine Beleuchtung aufweist.

9. Photobioreaktor nach einem der Ansprüche 1 bis 8, **dadurch gekennzeichnet, dass** zwei oder mehrere Systempumpen (4) vorgesehen sind.

10. Photobioreaktor nach einem der Ansprüche 1 bis 9, **dadurch gekennzeichnet, dass** mittels der Regelungseinheit (5) der Lichteintrag und/oder ein Heizen und/oder ein Kühlen des Reaktors, insbesondere abhängig von der Suspensionstemperatur, ermöglicht ist.

11. Photobioreaktor nach einem der Ansprüche 1 bis 10, **dadurch gekennzeichnet, dass** die Ernteeinrichtung (6) eine Zentrifuge oder einen Sedimentationsbehälter aufweist.

12. Verfahren zur Kultivation von Biomasse mittels Photosynthese, wobei die Biomasse Algen aufweist, wobei das Verfahren die folgenden Schritte aufweist:
- Bereitstellen einer wässrigen, Biomasse enthaltenden Kultursuspension in einem Photobioreaktor,
- Kultivieren der Biomasse durch Eintragen von Licht und ggf. Kohlendioxid und/oder Nährstoffen, wobei die Suspension in dem Photobioreaktor im Kreislauf gefördert wird,
- Abtrennen und Entfernen kultivierter Biomasse aus der Suspension und ggf. Entfernen von Sauerstoff und/oder sonstigen Stoffwechselprodukten der Biomasse, **gekennzeichnet durch** die Verwendung eines Photobioreaktors nach einem der Ansprüche 1 bis 11.

13. Verfahren nach Anspruch 12, **dadurch gekennzeichnet, dass** die Innenseiten der die Kultursuspension beinhaltenden Teile des Bioreaktors, insbesondere der Photosyntheseteil (2), und insbesondere Glasrohre (13) des Photosyntheseteils (2), periodisch durch die Mitförderung von Reinigungskörpern, insbesondere Reinigungsgranulat, abgereinigt werden.

14. Verfahren nach Anspruch 12 oder 13, **dadurch gekennzeichnet, dass** der Kultivationsschritt einem Regime künstlicher Beleuchtung unterworfen wird, wobei in Bezug auf den Tagesablauf eine Verlängerung des Tageslichts mit anschließender Dunkelphase (Nacht) und/oder in Bezug auf die Jahreszeiten ein kurzer Winter simuliert wird.

15. Verfahren nach einem der Ansprüche 12 bis 14, **dadurch gekennzeichnet, dass** der Kultivationsschritt einem Ernteregime unterworfen wird, wonach ca. alle 14 Tage ca. 1/3 des Volumens der Kultursuspension erneuert werden, insbesondere dadurch, dass der aus der Ernteeinrichtung (6) zurückerhaltene Klarlauf verworfen und durch frisches Kultivationsmedium ersetzt wird.

## Claims

1. Photobioreactor for cultivating biomass by means of photosynthesis, wherein the biomass has algae, wherein light and optionally carbon dioxide and/or food substances can be supplied inside the bioreactor to an aqueous culture suspension containing biomass, and wherein the photobioreactor has at least the following components:
- a photosynthesis member (2),
- a system or compensation container (3),
- a system pump (4),
- a control unit (5) and
- a separating and harvesting device (6),
wherein the photobioreactor is configured as a self-sufficient total system (1),
**characterised in that** the total system (1) is arranged in a container (7) or in a frame (23) which has the corresponding dimensions and which can be stacked accordingly.

2. Photobioreactor according to claim 1, **characterised in that** the total system (1) comprises all the necessary auxiliary units and/or storage containers for supplying or discharging media.

3. Photobioreactor according to claim 1 or 2, **characterised in that** the total system (1) is transportable.

4. Photobioreactor according to any one of claims 1 to 3, **characterised in that** the total system (1) is arranged in a 20-foot or 40-foot standard container (7) in accordance with ISO 668 or in a frame (23) which has the corresponding dimensions and which can be stacked accordingly, or **in that** the total system (1) can be arranged on a passenger vehicle or lorry trailer, a lorry swap body, a goods wagon, a float or a ship, wherein the container (7), the frame (23) or a receiving device of the type has a light-permeable roof and/or a shading device which can be opened and closed for the roof (energy shield) and/or walls (10) which reflect light and/or a floor (11) which reflects light.

5. Photobioreactor according to any one of claims 1 to 4, **characterised in that** an illumination device and/or a heating unit and/or a cooling device is/are associated with the reactor.

6. Photobioreactor according to any one of claims 1 to 5, **characterised in that** the photosynthesis member (2) is arranged in a resilient manner and/or **in that** the photosynthesis member (2) is divided into individual modules (12), where applicable wherein the modules (12) are inclined at an angle of from 0° to 90° with respect to the horizontal and/or **in that** two or more photosynthesis members (2) are provided.

7. Photobioreactor according to any one of claims 1 to 6, **characterised in that** the photosynthesis member (2) is constructed from glass tubes (13), in particular from borosilicate glass, and/or other pipe connections (17) are produced in the reactor from food-safe plastics material.

8. Photobioreactor according to any one of claims 1 to 7, **characterised in that** the system container (3) is encapsulated and/or has a heating unit and/or a cooling plant and/or a lighting unit.

9. Photobioreactor according to any one of claims 1 to 8, **characterised in that** two or more system pumps (4) are provided.

10. Photobioreactor according to any one of claims 1 to 9, **characterised in that**, by means of the control unit (5), the introduction of light and/or heating and/or cooling of the reactor is/are possible, in particular in accordance with the suspension temperature.

11. Photobioreactor according to any one of claims 1 to 10, **characterised in that** the harvesting device (6) has a centrifuge or a sedimentation container.

12. Method for cultivating biomass by means of photosynthesis, wherein the biomass has algae, wherein the method has the following steps:
- providing an aqueous culture suspension containing biomass in a photobioreactor,
- cultivating the biomass by introducing light and optionally carbon dioxide and/or food substances, wherein the suspension in the photobioreactor is conveyed in a circuit,
- separating and removing cultivated biomass from the suspension and where applicable removing oxygen and/or other metabolic products of the biomass, **characterised by** the use of a photobioreactor according to any one of claims 1 to 11.

13. Method according to claim 12, **characterised in that** the inner sides of the members of the bioreactor containing the culture suspension, in particular the photosynthesis member (2), and in particular glass tubes (13) of the photosynthesis member (2), are periodically cleaned by cleaning members, in particular cleaning granulate, also being conveyed.

14. Method according to claim 12 or claim 13, **characterised in that** the cultivation step is subjected to a regime of artificial lighting, wherein an increase of the daylight is simulated in relation to the course of the day with a subsequent dark phase (night), and/or a short winter is simulated in relation to the seasons.

15. Method according to any one of claims 12 to 14, **characterised in that** the cultivation step is subjected to a harvesting regime, according to which approximately every 14 days approximately 1/3 of the volume of the culture suspension is renewed, in particular **in that** the clear phase retained from the harvesting device (6) is discarded and replaced by fresh cultivation medium.

## Revendications

1. Photobioréacteur pour la culture de biomasse au moyen d'une photosynthèse, dans lequel la biomasse présente des algues, dans lequel de la lumière et, le cas échéant, du dioxyde de carbone et/ou des nutriments peuvent être amenés à une suspension de culture aqueuse contenant une biomasse à l'intérieur du bioréacteur, et dans lequel le photobioréacteur présente au moins les composants suivants :
- une partie de photosynthèse (2),
- un récipient de système ou de compensation (3),
- une pompe de système (4),
- une unité de régulation (5), et
- un dispositif de séparation ou de récolte (6),
le photobioréacteur étant conçu en tant que système global autosuffisant (1), **caractérisé en ce que** le système global (1) est agencé dans un conteneur (7) ou dans un cadre (23) présentant les dimensions correspondantes et pouvant être empilé de façon correspondante.

2. Photobioréacteur selon la revendication 1, **caractérisé en ce que** le système global (1) comprend l'ensemble des accessoires et/ou des récipients nécessaires pour les substances à amener ou à évacuer.

3. Photobioréacteur selon la revendication 1 ou 2, **caractérisé en ce que** le système global (1) peut être transporté.

4. Photobioréacteur selon l'une des revendications 1 à 3, **caractérisé en ce que** le système global (1) est agencé dans un conteneur standard de 20 pieds ou 40 pieds (7) conforme à la norme ISO 668 ou dans un cadre (23) présentant les dimensions correspondantes et pouvant être empilé de façon correspondante ou **en ce que** le système global (1) peut être agencé sur une remorque de voiture ou de camion, une caisse mobile de camion, un wagon de marchandises, un radeau ou un bateau, le conteneur (7), le cadre (23) ou le dispositif de réception similaire présentant un toit translucide et/ou un dispositif d'ombrage pouvant être déployé et rétracté pour le toit (écran énergétique) et/ou des parois (10) reflétant la lumière et/ou un fond (11) reflétant la lumière.

5. Photobioréacteur selon l'une des revendications 1 à 4, **caractérisé en ce qu'**un dispositif d'éclairage et/ou un chauffage et/ou un dispositif de refroidissement est adjoint au réacteur.

6. Photobioréacteur selon l'une des revendications 1 à 5, **caractérisé en ce que** la partie de photosynthèse (2) est montée de façon élastique et/ou **en ce que** la partie de photosynthèse (2) est répartie dans différents modules (12), les modules (12) étant le cas échéant inclinés dans un angle de 0° à 90° par rapport à l'horizontale, et/ou **en ce qu'**il est prévu deux parties de photosynthèse (2) ou davantage.

7. Photobioréacteur selon l'une des revendications 1 à 6, **caractérisé en ce que** la partie de photosynthèse (2) est constituée de tubes de verre (13), en particulier en verre borosilicate, et/ou des raccords de tuyaux divers (17) dans le réacteur sont réalisés en plastique adapté au contact alimentaire.

8. Photobioréacteur selon l'une des revendications 1 à 7, **caractérisé en ce que** le récipient de système (3) est encapsulé et/ou présente un chauffage et/ou une installation de refroidissement et/ou un éclairage.

9. Photobioréacteur selon l'une des revendications 1 à 8, **caractérisé en ce qu'**il est prévu deux pompes de système (4) ou davantage.

10. Photobioréacteur selon l'une des revendications 1 à 9, **caractérisé en ce que** l'apport de lumière et/ou un chauffage et/ou un refroidissement du réacteur, en particulier en fonction de la température de la suspension, est rendu possible au moyen de l'unité de régulation (5).

11. Photobioréacteur selon l'une des revendications 1 à 10, **caractérisé en ce que** le dispositif de récolte (6) présente une centrifugeuse ou un récipient de sédimentation.

12. Procédé de culture de biomasse au moyen d'une photosynthèse, dans lequel la biomasse présente des algues, le procédé présentant les étapes suivantes :
- fourniture d'une suspension de culture aqueuse contenant une biomasse à l'intérieur d'un photobioréacteur,
- culture de la biomasse au moyen de l'apport de lumière et, le cas échéant, de dioxyde de carbone et/ou de nutriments, la suspension étant déplacée dans un circuit dans le photobioréacteur,
- séparation et enlèvement de la biomasse cultivée de la suspension et, le cas échéant, enlèvement d'oxygène et/ou d'autres métabolites de la biomasse, **caractérisé par** l'utilisation d'un photobioréacteur selon l'une des revendications 1 à 11.

13. Procédé selon la revendication 12, **caractérisé en ce que** les côtés intérieurs des parties du bioréacteur contenant la suspension de culture, en particulier la partie de photosynthèse (2), et en particulier les tubes de verre (13) de la partie de photosynthèse (2), sont nettoyés périodiquement au moyen du déplacement simultané de corps de nettoyage, en particulier de granulés de nettoyage.

14. Procédé selon la revendication 12 ou 13, **caractérisé en ce que** l'étape de la culture est soumise à un régime d'éclairage artificiel, une prolongation de la lumière du jour par rapport au déroulement d'une journée, avec une phase sombre (nuit) consécutive, ou un hiver plus court par rapport aux saisons de l'année étant simulé.

15. Procédé selon l'une des revendications 12 à 14, **caractérisé en ce que** l'étape de la culture est soumise à un régime de récolte dans lequel environ un tiers du volume de la suspension de culture est renouvelé environ tous les 14 jours, en particulier **en ce que** l'écoulement clair récupéré à partir du dispositif de récolte (6) est rejeté et remplacé par un nouveau substrat de culture.
